Europäisches Patentamt

(19) European Patent Office

Office européen des brevets

(11) Numéro de publication : **0 300 878 B1**

## (12) FASCICULE DE BREVET EUROPEEN

(45) Date de publication du fascicule du brevet :
**06.11.91 Bulletin 91/45**

(51) Int. Cl.⁵ : **A61K 31/195, A61K 37/18**

(21) Numéro de dépôt : **88401808.6**

(22) Date de dépôt : **12.07.88**

(54) Compositions pharmaceutiques utiles pour le traitement de l'urémie.

(30) Priorité : **23.07.87 FR 8710406**

(43) Date de publication de la demande :
**25.01.89 Bulletin 89/04**

(45) Mention de la délivrance du brevet :
**06.11.91 Bulletin 91/45**

(84) Etats contractants désignés :
**AT BE CH DE FR GB IT LI LU NL SE**

(56) Documents cités :
**EP-A- 0 184 999**
**EP-A- 0 227 545**
**FR-A- 2 315 916**
**US-A- 4 352 814**
**R. VOIGT: "Lehrbuch der pharmazeutischen Technologie", 1975, pages 213-218, Verlag Volk und Gesundheit, Berlin, DE**

(73) Titulaire : **NB INTERNATIONAL TECHNOLOGIES**
**Avenue Nestlé 55**
**CH-1800 Vevey (CH)**

(72) Inventeur : **Bobee, Jean Marc**
**Résidence "Le Village" 3, Square des Poètes**
**F-91370 Verrières le Buisson (FR)**
Inventeur : **Melin, Christian**
**1, Square des Ecrivains**
**F-91370 Verrières le Buisson (FR)**

(74) Mandataire : **Thouret-Lemaitre, Elisabeth et al**
**SYNTHELABO Service Brevets 22, avenue Galilée**
**F-92352 LE PLESSIS ROBINSON CEDEX (FR)**

**Description**

La présente invention a pour objet des compositions pharmaceutiques utiles pour le traitement de l'urémie.

Des compositions constituées par des mélanges de sels formés par des α-céto-acides et des acides aminés basiques sont utiles en tant que suppléments nutritionnels pour le traitement de l'insuffisance rénale chronique.

Les sels d'acides α-aminés basiques et d'α-céto-analogues d'acides aminés essentiels à chaîne ramifiée ont été décrits dans les brevets FR-A,B-2419723 et FR-A,B-2454434.

Les compositions contenant les mélanges de plusieurs de ces sels et pouvant contenir également de la L-tyrosine, de la L-thréonine et de l'α-hydroxy-γ-méthylthiobutyrate de calcium ont été décrites dans la demande de brevet européen EP-A-0227545.

Les compositions contenant les mélanges de plusieurs sels d'acides α-aminés basiques et d'α-céto-analogues d'acides aminés essentiels à chaîne ramifiée et contenant également de la L-tyrosine, de la L-thréonine, du L-tryptophane et de l'α-hydroxy-γ-méthylthiobutyrate de calcium ont été décrites dans la demande de brevet européen EP-A-0295166.

Les compositions contenant les mélanges de plusieurs de ces sels et pouvant contenir également de la L-tyrosine, de la L-thréonine et de l'α-hydroxy-γ-méthylthiobutyrate de calcium et éventuellement du tryptophane peuvent être présentées sous forme de poudre ou de granulés que l'on peut dissoudre ou mettre en suspension dans de l'eau ou une boisson telle que du jus de pamplemousse ou d'orange.

Toutefois il est apparu que les compositions sous forme de poudre ou de granulés ne sont pas parfaitement stables à la température ambiante ; de plus elles ont un goût et une odeur désagréables qui indisposent les patients et limitent la compliance du traitement.

La Demanderesse a mis au point une formulation de comprimé enrobé gastrorésistant ou gastrosoluble pour les compositions qui seront décrites ci-après ; formulation qui présente une plus grande stabilité que la poudre ou les granulés, sans odeur et sans goût, rendant les compositions tout à fait acceptables par les patients.

La formulation de comprimé enrobé gastrosoluble ou gastrorésistant de l'invention comprend un noyau (constitué des principes actifs et de divers excipients appropriés) et un enrobage lui conférant la propriété d'être gastrosoluble ou gastrorésistant.

Le noyau comprend le mélange des principes actifs, un liant, un délitant, un agent absorbant d'eau et un agent lubrifiant.

Les quantités respectives des constituants du noyau sont les suivantes :

pour 100 g de noyau, la quantité d'agent liant peut aller de 0,5 à 5% en poids, la quantité de délitant peut aller de 2 à 20% en poids, la quantité d'agent absorbant d'eau peut aller de 0,5 à 15% en poids, la quantité d'agent lubrifiant peut aller de 0,5 à 3% en poids, le complément à 100 g étant constitué par le mélange des principes actifs.

Le mélange des principes actifs est un mélange de sels d'acides α-aminés basiques et d'α-céto-analogues d'acides aminés essentiels à chaîne ramifiée contenant tout ou partie des constituants suivants dans les proportions suivantes (exprimées en pourcentage en poids du mélange) :

| | |
|---|---|
| – α-céto-isovalérate (ou cétovaline) | 5-20 |
| – α-céto-isocaproate (ou cétoleucine) | 5-15 |
| – α-céto-β-méthyl-valérate (ou cétoisoleucine) | 5-15 |
| – L-ornithine | 15-25 |
| – L-lysine | 10-25 |
| – L-histidine | 1-10 |
| – L-thréonine | 1-10 |
| – L-tyrosine | 5-20 |
| – L-tryptophane | 0-5 |
| – D,L-α-hydroxy-γ- méthylthiobutyrate de calcium | 1-5 |

Les formulations de l'invention peuvent contenir de 250 à 1000 mg du mélange de principes actifs dans le noyau.

Le liant peut être un dérivé de la cellulose tel que l'hydroxy-propylméthyl-cellulose (HPMC) ou la carboxyméthylcellulose. Le délitant peut être un dérivé de polysaccharide tel le carboxyméthyl-amidon.

L'agent absorbant d'eau peut-être du gel de silice et l'agent lubrifiant du stéarate de magnésium.

L'enrobage peut représenter de 3 à 20% du poids du noyau. Il est réalisé par pulvérisation d'une solution organique contenant un agent filmogène, un ou plusieurs colorants et éventuellement un plastifiant auxquels peuvent être ajoutés divers agents de charge.

La solution d'enrobage peut contenir de 2 à 20% (poids/volu- me) d'agent filmogène, de 0 à 10% d'agents colorants, de 0 à 5% d'agent plastifiant et de 0 à 10% d'agents de charge, le complément à 100 ml étant constitué par un mélange en proportions appropriées d'un solvant de nature alcoolique et d'un solvant chloré ou d'une solution aqueuse.

La quantité de solution d'enrobage pulvérisée pour 100 g de noyau varie de 50 à 150 ml en fonction de la nature chimique de l'agent filmogène utilisé.

L'agent filmogène peut être gastrosoluble ou gastrorésistant. A titre d'exemple l'agent filmogène gastrosoluble peut être un polymérisat cationique de diméthylaminoéthylméthacrylate et d'autres esters neutres d'acide méthacrylique ou l'hydroxypropylméthylcellulose.

L'agent filmogène gastrorésistant peut être un phtalate d'hydroxypropylméthylcellulose (HPMC).

L'agent colorant peut être une suspension organique de dioxyde de titane et le plastifiant du phtalate de diéthyle ou ou un monoglycéride acétylé.

Les agents de charge peuvent être du stéarate de magnésium ou du talc.

Le solvant de la solution d'enrobage est préférentiellement constitué d'un mélange 50/50 de chlorure de méthylène et d'éthanol.

Les exemples suivants illustrent l'invention :

Exemple 1 :        Comprimé enrobé gastrosoluble

Noyau :

| Principes actifs (P.A.) | Composition centésimale théorique (en g) | Composition d'un comprimé dosé à 770 mg de P.A. (en mg) |
|---|---|---|
| L-tyrosine | 18,006 | 150,71 |
| L-thréonine | 8,879 | 74,31 |
| L-tryptophane | 0,497 | 4,16 |
| α-céto-ß-méthyl-valérate de L-ornithine | 18,245 | 152,71 |
| α-céto-isovalérate de L-ornithine | 8,636 | 72,28 |
| α-céto-isocaproate de L-Lysine | 19,223 | 160,89 |
| α-céto-isovalérate de L-lysine | 9,123 | 76,35 |
| α-céto-isocaproate de L-histidine, H₂O | 6,027 | 50,44 |
| D,L- α-hydroxy-γ-méthyl-thiobutyrate de Ca | 3,364 | 28,15 |

Excipients du noyau

| | | |
|---|---|---|
| Carboxyméthylamidon (PRIMOJEL MD) ® | 5 | 41,85 |
| Gel de silice | 2 | 16,74 |
| Stéarate de Magnésium | 1 | 8,37 |
| | 100 | 836,96 |

Solution d'enrobage

EUDRAGIT E 100 * ®       5 g

Talc                      3,5 g

Stéarate de Magnésium     1 g

colorant (SEPISPERSE ®

BLANC K 7001)            5 g

solvants : Ethanol 50

            $CH_2 Cl_2$ 50

                  q.s.p.    100 ml

NB : 60 ml de solution d'enrobage pour 100 g de comprimé (soit 3 % d'agent filmogène par rapport au poids de noyau).

* EUDRAGIT E = polymérisat cationique de diméthylaminoéthyl-méthacrylate et d'autres esters neutres d'acide méthacrylique.

Exemple 2    Comprimé enrobé gastrorésistant

Noyau

| Principes actifs | Composition centésimale théorique (en g) | Composition d'un comprimé dosé à 850 mg de P.A. (en mg) |
|---|---|---|
| L tyrosine | 18,17 | 167,87 |
| L thréonine | 8,96 | 82,78 |
| α-céto-ß-méthyl-valé-rate de L-ornithine | 18,42 | 170,18 |
| α-céto-isovalérate de L-ornithine | 8,72 | 80,56 |
| α-céto-isocaproate de L-lysine | 19,40 | 179,24 |
| α-céto-isovalérate de L-lysine | 9,21 | 85,09 |
| α-céto-isocaproate de L-histidine | 5,72 | 52,85 |
| D,L-α-hydroxy-γ-méthyl thiobutyrate de Ca | 3,40 | 31,41 |

Excipients du noyau :

| | | |
|---|---|---|
| Carboxyméthylamidon (PRIMOJEL ND) ® | 5 | 46,20 |
| Gel de silice | 2 | 18,48 |
| Stéarate de Magnésium | 1 | 9,24 |
| | 100 | 923,90 |

6

<u>Solution d'enrobage</u>

Phtalate d'HPMC (HP 55
- SEPPIC) ®                10             92,39

Phtalate de diéthyle       2            18,48

Colorant (SEPISPERSE ® JAUNE
AP 3012)                 5            23,10

Solvants :

. Ethanol 50                        –

. $CH_2$ $Cl_2$ 50

q.s.p.   100 ml

NB : 100 ml de solution d'enrobage pour 100 g de comprimé
(soit 10 % d'agent filmogène par rapport au poids de noyau).

**Revendications**

**Revendications pour les Etats contractants suivants : AT, BE, CH, DE, FR, GB, IT, LI, LU, NL, SE.**

1. Compositions pharmaceutiques, contenant un mélange de plusieurs sels d'acides α-aminés basiques et d'α-céto-analogues d'acides aminés essentiels à chaîne ramifiée, caractérisées par le fait qu'elles sont présentées sous forme de comprimé enrobé gastrorésistant ou gastrosoluble.

2. Compositions pharmaceutiques selon la revendication 1, contenant, en plus du mélange de sels, de la L-tyrosine, de la L-thréonine, et du D,L-α-hydroxy-y-méthylthio-butyrate de calcium et éventuellement du L-tryptophane.

3. Compositions pharmaceutiques selon la revendication 1 ou la revendication 2, caractérisées par le fait que la formulation de comprimé enrobé gastrosoluble ou gastrorésistant est constituée par un noyau comprenant le mélange de principes actifs (mélange de sels et autres constituants éventuels), un liant, un délitant, un agent absorbant d'eau et un agent lubrifiant et par un enrobage gastrosoluble ou gastrorésistant formé à partir d'une solution contenant un agent filmogène, un ou plusieurs colorants et éventuellement un plastifiant auxquels peuvent être ajoutés divers agents de charge.

4. Compositions pharmaceutiques selon l'une quelconque des revendications 1 à 3, caractérisées par le fait que le solvant de la solution d'enrobage est un mélange de chlorure de méthylène et d'éthanol.

5. Compositions pharmaceutiques selon l'une quelconque des revendications 1 à 4, caractérisées par le fait que l'agent liant est un dérivé de la cellulose tel que l'hydroxy-propylméthyl-cellulose ou la carboxyméthylcellulose.

6. Compositions pharmaceutiques selon l'une quelconque des revendications 1 à 5, caractérisées par le fait que l'agent délitant est un dérivé de polysaccharide tel que le carboxyméthylamidon.

7. Compositions pharmaceutiques selon l'une quelconque des revendications 1 à 6, caractérisées par le fait que l'agent absorbant d'eau est le gel de silice.

8. Compositions pharmaceutiques selon l'une quelconque des revendications 1 à 7, caractérisées par le fait que l'agent lubrifiant est le stéarate de magnésium.

9. Compositions pharmaceutiques selon l'une quelconque des revendications 1 à 8, caractérisées par le fait que l'agent filmogène gastrosoluble est un polymérisat cationique de diméthylaminoéthylméthacrylate et d'autres esters neutres d'acide méthacrylique.

10. Compositions pharmaceutiques selon l'une quelconque des revendications 1 à 8, caractérisées par le fait que l'agent filmogène gastrorésistant est le phtalate d'hydroxypropylméthylcellulose.

11. Composition pharmaceutique selon l'une quelconque des revendications 1 à 10, caractérisée en ce qu'elle contient les principes actifs suivants : cétovaline, cétoleucine, céto-isoleucine, L-ornithine, L-lysine, L-histidine, L-thréonine, L-tyrosine et D,L-α-hydroxy-γ-méthylthiobutyrate de calcium.

12. Composition pharmaceutique selon l'une quelconque des revendications précédentes 1 à 10, caractérisée en ce qu'elle contient les principes actifs suivants : L-tyrosine, L-thréonine, α-céto-β-méthyl-valérate de L-ornithine, α-cétoisocaproate de L-lysine, α-céto-isovalérate de L-lysine, α-céto-isocaproate de L-histidine, H$_2$O, D,L-α-hydroxy-γ-méthyl-thiobutyrate de Ca.

## Claims

**Claims for the following Contracting States : AT, BE, CH, DE, FR, GB, IT, LI, LU, NL, SE.**

1. Pharmaceutical compositions containing a mixture of several salts of basic α-amino acids and of α-keto analogues of branched-chain essential amino acids, characterised in that they are presented in the form of a gastroresistant or gastrosoluble coated tablet.

2. Pharmaceutical compositions according to Claim 1, containing in addition to the mixture of salts, L-tyrosine, L-threonine and calcium DL-α-hydroxy-γ-methyl-thiobutyrate, and optionally L-tryptophan.

3. Pharmaceutical compositions according to Claim 1 or Claim 2, characterised in that the gastrosoluble or gastroresistant coated tablet formulation consists of a core comprising the mixture of active principles (mixture of salts and other possible constituents), a binder, a disintegrating agent, a water-absorbing agent and a lubricant, and of a gastrosoluble or gastroresistant coating formed from a solution containing a film-forming agent, one or more colourings and optionally a plasticizer, to which various filler ingredients may be added.

4. Pharmaceutical compositions according to any one of Claims 1 to 3, characterised in that the solvent of the coating solution is a mixture of methylene chloride and ethanol.

5. Pharmaceutical compositions according to any one of Claims 1 to 4, characterised in that the binding agent is a cellulose derivative such as hydroxypropylmethyl-cellulose or carbomethylcellulose.

6. Pharmaceutical compositions according to any one of Claims 1 to 5, characterised in that the disintegrating agent is a polysaccharide derivative such as carboxymethylstarch.

7. Pharmaceutical compositions according to any one of Claims 1 to 6, characterised in that the water-absorbing agent is silica gel.

8. Pharmaceutical compositions according to any one of Claims 1 to 7, characterised in that the lubricant is magnesium stearate.

9. Pharmaceutical compositions according to any one of Claims 1 to 8, characterised in that the gastrosoluble film-forming agent is a cationic polymerisate of dimethylaminoethyl methacrylate and of other neutral esters of methacrylic acid.

10. Pharmaceutical compositions according to any one of Claims 1 to 8, characterised in that the gastroresistant film-forming agent is hydroxypropylmethyl-cellulose phthalate.

11. Pharmaceutical composition according to any one of Claims 1 to 10, characterised in that it contains the following active principles : ketovaline, ketoleucine, ketoisoleucine, L-ornithine, L-lysine, L-histidine, L-threonine, L-tyrosine and calcium DL-α-hydroxy-γ-methylthiobutyrate.

12. Pharmaceutical composition according to any one of the preceding Claims 1 to 10, characterised in that it contains the following active principles : L-tyrosine, L-threonine, L-ornithine α-keto-β-methylvalerate, L-ornithine α-ketoisovalerate, L-lysine α-ketoisocaproate, L-lysine α-ketoisovalerate, L-histidine α-ketoisocaproate, H$_2$O, Ca DL-α-hydroxy-γ-methylthiobutyrate.

## Patentansprüche

**Patentansprüche für folgende Vertragsstaaten AT, BE, CH, DE, FR, GB, IT, LI, LU, NL, SE.**

1. Pharmazeutische Zusammensetzungen, die eine Mischung mehrerer Salze von basischen alpha-Aminosäuren und alpha-Keto-Analoga verzweigtkettiger, essentieller Aminosäuren enthalten, dadurch gekennzeichnet, daß sie in Form umhüllter, gastroresistenter oder gastrolöslicher Tabletten vorliegen.

2. Pharmazeutische Zusammensetzungen nach Anspruch 1, die zusätzlich zu der Salzmischung L-Tyrosin, L-Threonin und Calcium-D,L-alpha-Hydroxy-gamma-methylthiobutyrat sowie gegebenenfalls L-Tryptophan enthalten.

3. Pharmazeutische Zusammensetzungen nach Anspruch 1 oder Anspruch 2, dadurch gekennzeichnet, daß die Formulierung der umhüllten, gastrolöslichen oder gastroresistenten Tablette aus einem Kern und einer gastrolöslichen oder gastroresistenten Umhüllung besteht, wobei der Kern die Mischung der Wirkstoffe (Mischung der Salze und anderer wahlweiser Bestandteile), ein Bindemittel, ein Zerfallmittel, ein Wasserabsorptionsmittel und ein Schmiermittel enthält und die Umhüllung aus einer ein filmbildendes Mittel, einen oder mehrere Farbstoffe und gegebenenfalls einen Weichmacher enthaltenden Lösung, der verschiedene Zusatzstoffe zugesetzt sein können, hergestellt ist.

4. Pharmazeutische Zusammensetzungen nach einem der Ansprüche 1 bis 3, dadurch gekennzeichnet, daß das Lösungsmittel der Umhüllungslösung eine Mischung von Methylenchlorid und Ethanol ist.

5. Pharmazeutische Zusammensetzungen nach einem der Ansprüche 1 bis 4, dadurch gekennzeichnet, daß das Bindemittel ein Zellulosederivat, wie Hydroxypropylmethylzellulose oder Carboxymethylzellulose ist.

6. Pharmazeutische Zusammensetzungen nach einem der Ansprüche 1 bis 5, dadurch gekennzeichnet, daß das Zerfallmittel ein Polysaccharidderivat, wie Carboxymethylstärke, ist.

7. Pharmazeutische Zusammensetzungen nach einem der Ansprüche 1 bis 6, dadurch gekennzeichnet, daß das Wasserabsorptionsmittel Silikagel ist.

8. Pharmazeutische Zusammensetzungen nach einem der Ansprüche 1 bis 7, dadurch gekennzeichnet, daß das Schmiermittel Magnesiumstearat ist.

9. Pharmazeutische Zusammensetzungen nach einem der Ansprüche 1 bis 8, dadurch gekennzeichnet, daß das gastrolösliche filmbildende Mittel ein kationisches Polymerisat von Dimethylaminoethylmethacrylat und anderen neutralen Methacrylsäureestern ist.

10. Pharmazeutische Zusammensetzungen nach einem der Ansprüche 1 bis 8, dadurch gekennzeichnet, daß das gastroresistente filmbildende Mittel Hydroxypropylmethylzellulose-Phthalat ist.

11. Pharmazeutische Zusammensetzung nach einem der Ansprüche 1 bis 10, dadurch gekennzeichnet, daß sie folgende Wirkstoffe enthält : Ketovalin, Ketoleucin, Ketoisoleucin, L-Ornithin, L-Lysin, L-Histidin, L-Threonin, L-Tyrosin und Calcium-D,L-alpha-Hydroxy-gamma-methylthiobutyrat.

12. Pharmazeutische Zusammensetzung nach einem der vorhergehenden Ansprüche 1 bis 10, dadurch gekennzeichnet, daß sie folgende Wirkstoffe enthält : L-Tyrosin, L-Threonin, L-Ornithin-alpha-Keto-beta-methylvalerat, L-Ornithin-alpha-Ketoisovalerat, L-Lysin-alpha-Ketoisocaproat, L-Lysin-alpha-Ketoisovalerat, L-Histidin-alpha-Ketoisocaproat, $H_2O$, Ca-D,L-alpha-Hydroxygamma-methylthiobutyrat.